(19) Europäisches Patentamt
European Patent Office
Office européen des brevets

(11) **EP 1 352 543 B1**

(12) **FASCICULE DE BREVET EUROPEEN**

(45) Date de publication et mention
de la délivrance du brevet:
**11.08.2010 Bulletin 2010/32**

(51) Int Cl.:
***H04R 29/00*** *(2006.01)*

(21) Numéro de dépôt: **01907628.0**

(86) Numéro de dépôt international:
**PCT/FR2001/000163**

(22) Date de dépôt: **18.01.2001**

(87) Numéro de publication internationale:
**WO 2002/058434 (25.07.2002 Gazette 2002/30)**

(54) **GENERATEUR DE SIGNAUX AUDITIFS DESTINES A DES PERSONNES ATTEINTES D'ACOUPHENES**

ERZEUGER VON AKUSTISCHEN SIGNALEN FÜR PERSONEN MIT TINNITUSSTÖRUNGEN

AUDITORY SIGNAL GENERATOR FOR PEOPLE SUFFERING FROM TINNITUS

(84) Etats contractants désignés:
**AT BE CH CY DE DK ES FI FR GB GR IE IT LI LU MC NL PT SE TR**

(43) Date de publication de la demande:
**15.10.2003 Bulletin 2003/42**

(73) Titulaire: **CENTRE NATIONAL DE LA RECHERCHE SCIENTIFIQUE (CNRS)**
**75794 Paris Cedex 16 (FR)**

(72) Inventeurs:
• **MICHEYL, Christophe**
**Belmont MA 02478 (US)**
• **NORENA, Arnaud**
**33140 Pont de la Maye/VILLENAVE D'ORNON (FR)**

(74) Mandataire: **Novagraaf Technologies**
**122 rue Edouard Vaillant**
**92593 Levallois-Perret Cedex (FR)**

(56) Documents cités:
**WO-A-97/23117      DE-U- 29 706 812**
**US-A- 4 222 393**

**Description**

La présente invention est relative à un procédé de programmation d'un appareil permettant de générer des signaux auditifs destinés à l'écoute par des personnes souffrant d'acouphène. Elle vise également un appareil mettant en oeuvre un tel procédé et dont les caractéristiques des signaux générés sont adaptés de façon spécifique aux caractéristiques audiologiques d'un individu de manière à favoriser la disparition ou l'atténuation des acouphènes chez cet individu.

Par acouphène, on désigne la perception de bruits dans l'oreille ou dans la tête qui ne correspondent à aucun signal acoustique dans l'environnement. La nature de ces bruits et l'intensité avec laquelle ils se manifestent peuvent varier selon le cas. Généralement, la gêne occasionnée par l'acouphène peut être importante pour le sujet ; elle peut devenir invalidante et entraîner des troubles psychologiques graves, notamment des perturbations de l'humeur et du sommeil. De plus, la plupart des personnes qui souffrent d'acouphène ont également une perte auditive plus ou moins grave.

De façon classique, une personne atteinte d'acouphène se voit proposer différents types de traitements dont les effets réels ne sont pas toujours concluants. La pharmacologie représente le mode de traitement le plus commun mais aucune des substances prescrites n'a d'effet bénéfique démontré et certaines d'entre elles ont même des effets négatifs, allant jusqu'à aggraver la gêne occasionnée par l'acouphène.

Il existe également des appareils masqueurs d'acouphène qui sont des systèmes délivrant un signal sonore dans l'oreille du patient, de manière à rendre l'acouphène inaudible, soit de façon directe et immédiate, par masquage perceptif, soit de façon progressive, en vertu d'un processus d'habituation physiologique.

Les masqueurs d'acouphènes proposés à ce jour posent plusieurs problèmes :

-   les possibilités de génération de signaux de ces systèmes sont très limitées. En effet, dans la plupart des cas, ils ne permettent de produire qu'un seul type de signaux avec des caractéristiques spectrales très simples ce qui limite considérablement les possibilités d'adaptation au cas particulier de chaque patient.
-   de plus, les masqueurs existants se présentent généralement sous la forme de prothèses auditives. Ce type d'appareil rebute nombre de patients acouphéniques, surtout les plus jeunes, qui ne présentent pas de perte auditive suffisante pour nécessiter le port d'une prothèse auditive, et ne veulent pas donner l'impression qu'ils sont atteints d'un handicap.
-   enfin, l'adaptabilité insuffisante de ces masqueurs au cas particulier de chaque personne acouphénique entraîne leur inefficacité et aggrave même, dans certains cas, la sévérité du trouble.

La présente invention vise donc à palier ces inconvénients en proposant un procédé de programmation d'un appareil générateur de signaux auditifs dont les caractéristiques physiques sont adaptées au cas particulier d'une personne acouphénique : leur écoute prolongée doit entraîner la disparition définitive ou temporaire, ou tout au moins atténuer l'acouphène et la gêne qui lui est associée.

A cet effet, l'invention se rapporte à un procédé selon la revendication 1.

L'invention vise également un générateur de signaux auditifs selon la revendication 7.

Le document US-A-4222393 décrit un procédé pour réduire voire de supprimer les acouphènes. Dans ce procédé, un patient souffrant d'acouphènes est soumis à des sons de différentes hauteurs pour permettre au patient d'identifier un son ayant la même hauteur que le son perçu du fait de ses acouphènes. Le patient est ensuite soumis à un générateur de signaux auditifs produisant des fréquences pour masquer l'acouphène.

Le document DE 29706812 enseigne également un appareil programmé pour le traitement des acouphènes.

Le document WO-A-9723117 décrit de façon générale un procédé pour appliquer des signaux auditifs à patient à partir de mesures auditives sur ce patient. Le document WO-A-9723117 ne concerne pas le domaine technique des procédés permettant de réduire voire de supprimer les acouphènes.

Aucun des documents de l'état de la technique n'enseigne un procédé permettant une réduction satisfaisante des acouphènes.

L'invention a pour objet de fournir des signaux auditifs particuliers permettant une réduction satisfaisante des acouphènes.

D'autres caractéristiques et avantages de la présente invention ressortiront de la description faite ci-après, en référence aux dessins annexés qui en illustrent un exemple de réalisation dépourvu de tout caractère limitatif. Sur les figures :

-   la figure 1 représente les différentes étapes d'un procédé de programmation d'un générateur de signaux auditifs, selon l'invention ;
-   la figure 2 représente schématiquement la forme calculée de l'enveloppe spectrale du signal adapteur-équaliseur selon l'invention ;
-   la figure 3 est une représentation à plusieurs niveaux du système auditif des réponses neuronales évoquées par un signal adapteur-équaliseur ;
-   la figure 4 est une représentation à plusieurs niveaux du système auditif des réponses neuronales évoquées par un bruit blanc ;
-   les figures 5a, 5b et 5c représentent l'organisation tonotopique des neurones centraux respectivement chez un sujet normo-entendant, un sujet acouphénique présentant une perte auditive et le même sujet après l'écoute d'un signal adapteur-équaliseur.

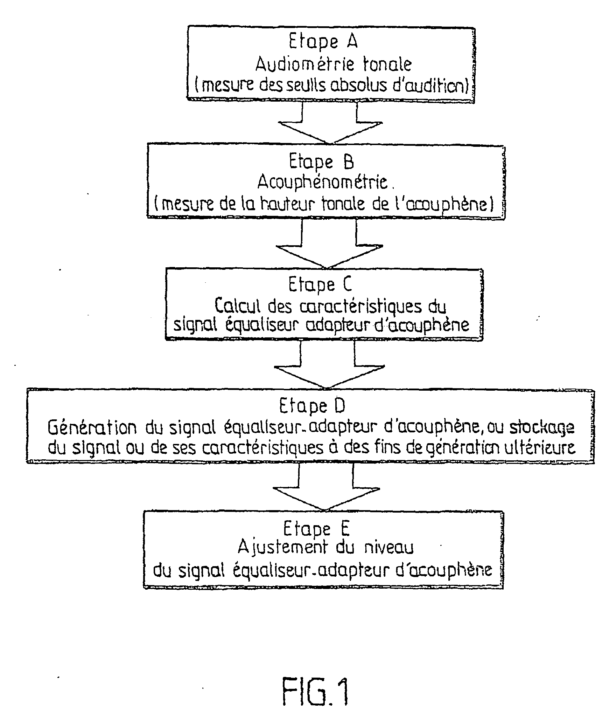

Selon un mode préféré de mise en oeuvre du procédé selon l'invention, ledit procédé de programmation d'un générateur de signaux sonores comprend 4 grandes phases opératoires distinctes et en particulier une étape intermédiaire entre les étapes principales a et b correspondant à la mesure de la fréquence perçue de l'acouphène (figure 1) :

> P our réaliser la première étape et l'étape intermédiaire, on utilise des premiers et seconds moyens qui peuvent être identiques. Notamment, ces premiers et seconds moyens peuvent comporter un générateur de sons purs, des atténuateurs logarithmiques, un préamplificateur de casque d'écoute et un casque d'écoute.

Une première phase opératoire (cf. étape A) consiste en la mesure des seuils absolus d'audition. Ces seuils, définis comme étant la plus petite intensité sonore que le sujet puisse percevoir, sont mesurés au moyen de sons purs de différentes fréquences dont on fait varier l'intensité en fonction des réponses du sujet, lequel a pour tâche d'indiquer au moyen d'un dispositif de réponse (de type bouton poussoir ou autre) s'il entend au non ces sons. Pour ce faire, un son intermittent de fréquence et d'intensité données est émis par des premiers moyens dans l'une des oreilles du sujet ; le sujet indique alors s'il a entendu.ou non ce son. Le son émis peut être composé, par exemple, de trois bouffées tonales successives, chaque bouffée ayant une durée de 400 millisecondes, incluant avantageusement des rampes cosinus-carré de 20 millisecondes ; les trois bouffées étant séparées par un intervalle de 200 ms. La fréquence choisie peut être de 2000 Hz par exemple, et le niveau de 60 décibels.

Si le son a été entendu par le sujet, le niveau du signal est réduit d'une certaine quantité x en décibels (par exemple, 10 dB). En revanche, si ce même son n'a pas été entendu, le niveau est augmenté d'une quantité y en décibels (par exemple, 20 dB). Le son est alors à nouveau présenté et la procédure se répète, les quantités x et y étant progressivement réduites.

Le critère d'arrêt de la procédure consiste soit en l'atteinte d'un niveau minimum, soit en la réalisation d'un nombre suffisant et préfixé d'inversions autour du seuil estimé, soit en l'atteinte d'une valeur minimum préfixée.

Lorsqu'une estimation de ce seuil absolu d'audition à une fréquence donnée est obtenue, la mesure du seuil à une fréquence suivante commence. Cette fréquence de test suivante est déterminée au moyen d'un dispositif identique au précédent et suivant une procédure dichotomique selon les règles suivantes :

- toutes les fréquences à l'octave entre 500 et 16000 Hz (à savoir, 500, 1000, 2000, 4000, 8000 et 16000 Hz) sont testées, dans un ordre pseudo-aléatoire de façon à éviter les effets d'habituation ou de fatigue.
- si au terme de ces mesures, aucune augmentation relative de seuil (c'est à dire, une différence de plus de 10 dB entre les seuils mesurés à l'une ou à l'autre des différentes fréquences testées) n'est observée, des mesures de seuil sont effectuées aux fréquences intermédiaires (demi-octaves), d'abord autour de 4000 Hz, puis aux fréquences supérieures et aux fréquences inférieures.
- si une augmentation relative de seuil est observée par le sujet à une fréquence donnée, des mesures de seuils sont effectuées plus précisément autour de cette fréquence, en commençant par tester les fréquences supérieures et inférieures selon un écart d'une demi-octave, puis ce pas fréquentiel est réduit et la procédure de mesure reprend aux fréquences intermédiaires.
- le critère d'arrêt de la procédure consiste en l'atteinte d'une valeur de pas fréquentiel minimum ou en un autre critère permettant d'attester qu'un niveau de précision suffisamment élevé a été atteint. Les seuils absolus d'audition sont successivement mesurés dans les deux oreilles au moyen de la phase opératoire décrite ci-dessus.

Une deuxième phase opératoire (cf. étape B) consiste en la mesure de la fréquence perçue de l'acouphène. Cette mesure de la hauteur de l'acouphène s'effectue soit au moyen d'une procédure d'encadrement dichotomique, soit suivant une procédure d'ajustement. Dans le premier cas, un son intermittent à une fréquence donnée est émis par des seconds moyens dans l'une au moins des oreilles du sujet. Par exemple, ce son est composé de trois bouffées de 1 seconde, séparées chacune par 1 seconde de silence et à une fréquence de 2000 Hz. La tâche du sujet est d'indiquer au moyen d'une interface de type boîtier réponse si ce son est plus aigu, plus grave ou bien de même tonalité que l'acouphène. Le sujet aura également la possibilité d'entendre à nouveau le même son avant de se prononcer.

Si le son émis est plus aigu, la fréquence du son test est divisée par un certain facteur. En revanche, si ce même son est plus grave, la fréquence du son test est multipliée par le même facteur. Puis la procédure se répète, le facteur étant réduit chaque fois que la réponse du sujet s'inverse (et augmenté chaque fois que le sujet donne la même réponse plus de deux fois de suite) jusqu'à ce que la valeur de ce facteur atteigne une valeur minimum préfixée ou bien que le sujet indique que le son test est de même tonalité que l'acouphène.

Selon une caractéristique avantageuse du procédé selon l'invention et par incidence d'un générateur mettant en oeuvre ledit procédé, des troisièmes moyens permettent d'ajuster, à chaque fréquence, le niveau du son test sur la base des résultats de la mesure des seuils effectués à la première étape, de sorte que ce niveau se maintienne entre 5 et 10 dB au-dessus du seuil absolu et soit ainsi toujours approximativement de même intensité perçue que l'acouphène. Cet ajustement a pour avantage de faciliter de façon substantielle la mesure de la hauteur tonale de l'acouphène. En effet, l'intensité étant un facteur crucial pour la caractérisation de la hauteur de l'acouphène, en neutralisant l'effet de celle-ci, il est plus aisé de localiser la fréquence qui correspond à la hauteur de l'acouphène.

Après qu'une première estimation de la fréquence de l'acouphène a été obtenue, d'autres mesures successives sont effectuées jusqu'à atteindre un critère de reproductibilité suffisante de la mesure. Un tel critère consiste par exemple, en l'obtention d'un intervalle de confiance à 95% ayant une étendue égale ou inférieure à une étendue minimum requise et prédéfinie autour de la hauteur tonale estimée de l'acouphène.

Chez les sujets qui indiquent percevoir leur acouphène dans une oreille, la mesure de la hauteur de l'acouphène selon la phase opératoire décrite ci-dessus est effectuée successivement avec ledit son intermittent présenté dans cette oreille (appariement ipsilatéral), puis dans l'oreille opposée (appariement controlatéral). Chez les sujets qui indiquent percevoir un acouphène centré dans l'espace intracrânien, le son d'appariement est présenté simultanément dans les deux oreilles, avec la même phase. Chez les sujets qui disent percevoir un acouphène localisé dans les deux oreilles, l'appariement de l'acouphène de chaque oreille est effectué en présentant le son test dans la même oreille (appariement ipsilatéral).

Au terme de cette étape de mesure de hauteur tonale de l'acouphène, il est vérifié que cette hauteur tombe dans, ou à proximité d'une zone fréquentielle dans laquelle les seuils absolus d'audition sont augmentés.

La troisième phase opératoire (cf. étape C) consiste à déterminer sur la base des mesures obtenues, les caractéristiques d'un signal adapteur-équaliseur adapté au cas particulier du sujet.

Ainsi, à partir des données mesurées dans les étapes précédentes, l'enveloppe spectrale encore appelée caractéristique du signal est calculée à l'aide d'algorithmes, de façon à compenser la ou les pertes d'audition mesurées à la première étape. De façon schématique, cette enveloppe correspond au négatif (sur l'échelle des ordonnées qui représente un gain en intensité) de l'audiogramme tonal mesuré à la première étape : l'amplitude de cette enveloppe est plus élevée dans les régions dans lesquelles les seuils absolus d'audition sont les plus élevés.

Les formules algorithmiques pour le calcul de cette enveloppe du signal adapteur-équaliseur à partir de l'audiogramme tonal sont les suivantes :

Soient Ai les seuils absolus d'audition (en décibels) aux différentes fréquences Fi auxquelles ces seuils ont été testés, soient Aj les seuils absolus d'audition aux fréquences Fj (estimés par interpolation à partir des Ai mesurés) avec j appartenant au groupe des entiers [1 ; N] où N est le nombre de points dans la fenêtre spectrale, et soit $Fj = j * (Fe / N)$, Fe étant la fréquence d'échantillonnage. L'enveloppe spectrale du signal adapteur-équaliseur en chaque point j de la fenêtre spectrale est alors calculée grâce à la formule mathématique suivante :

$$Ej = B + Mj - Qr(Aj)$$

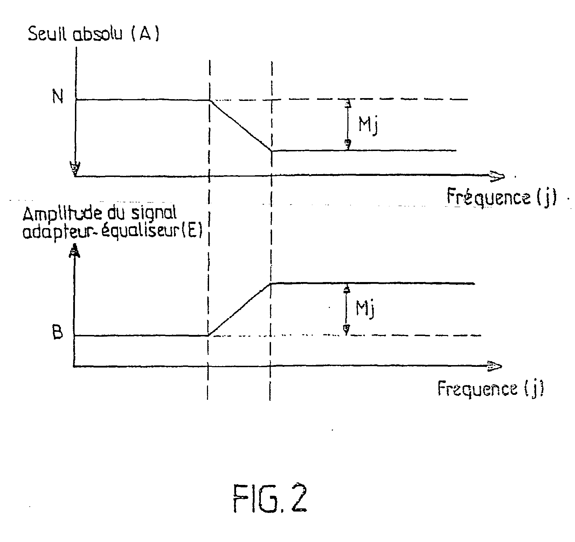

où : B représente le niveau minimal du signal adapteur-équaliseur ; $Mj = N - Aj$ où N est la moyenne des seuils minimaux obtenus à l'audiométrie tonale ; et $Qr(Aj)$ est un facteur de correction introduit pour tenir compte des effets de la compression cochléaire et de la réduction de sélectivité fréquentielle sur la quantité d'excitation produite par le signal dans le système auditif. La figure 2 représente schématiquement l'enveloppe spectrale du signal adapteur-équaliseur ainsi calculé à partir des seuils absolus d'audition.

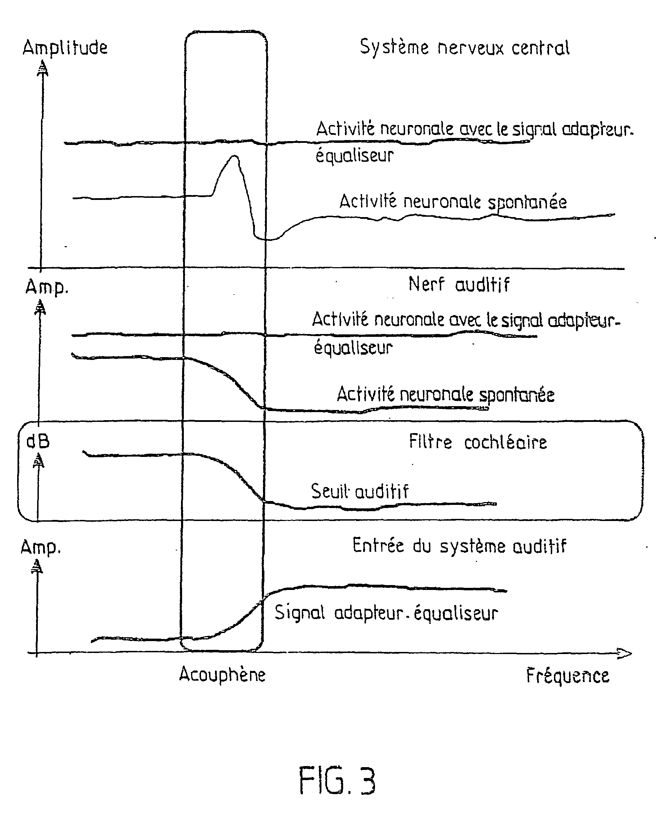

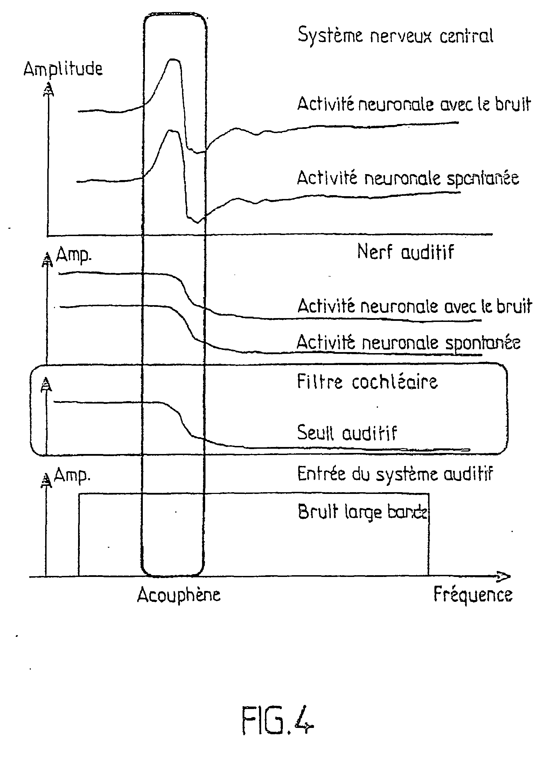

Ainsi, et selon une caractéristique avantageuse du procédé selon l'invention, les caractéristiques dudit signal auditif compensent le plus exactement possible la perte auditive du sujet, afin d'éliminer les contrastes spectraux dans l'activité du système auditif qui sont occasionnés par la perte auditive. En d'autres termes, ce signal vise à faire disparaître les discontinuités dans l'activité des neurones à travers les fréquences. La figure 3 représente, à plusieurs niveaux du système auditif, des réponses neuronales évoquées par un tel signal adapteur-équaliseur en présence d'une perte auditive. En comparaison, la figure 4 représente le même schéma de réponses neuronales évoquées par un bruit blanc utilisé dans les appareils masqueurs d'acouphène proposés à ce jour sur le marché : avec un signal adapteur-équaliseur calculé et programmé selon l'invention et adapté au cas particulier de chaque sujet, celui-ci est sensiblement plat et constant, tandis qu'avec un signal de type bruit blanc, l'activité neuronale présente toujours un pic au niveau de la fréquence d'acouphène, lequel est interprété comme un son par le système nerveux central.

Une caractéristique de la présente invention par rapport à ces appareils (ainsi que ceux qui, de façon plus générale, délivrent un signal dont les caractéristiques ne sont pas adaptées à la perte auditive de l'individu) est que les caractéristiques du signal sont déterminées sur la base d'une mesure préalable de la forme précise de la perte auditive du sujet.

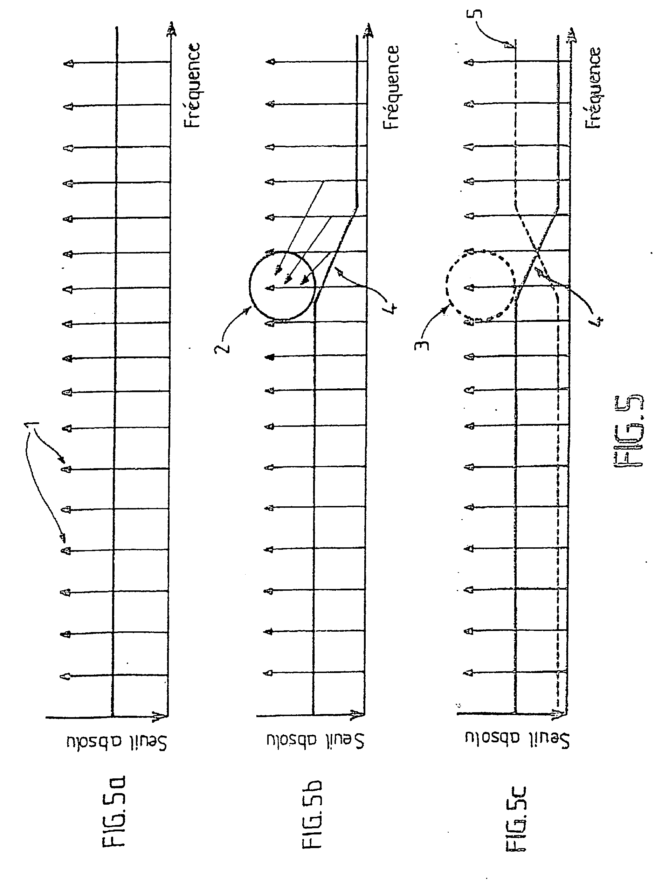

En outre, seul un tel signal spécifiquement adapté à l'audition des sujets, soumis pendant une période suffisamment longue au système auditif, entraîne au sein de celui-ci une inversion des mécanismes de plasticité cérébrale successifs à l'apparition de la perte auditive et associés à l'émergence et au maintien de l'acouphène. Ainsi, les figures 5a à 5c représentent l'organisation tonotopique des neurones centraux chez un sujet normo-entendant (figure 5a), chez un sujet acouphénique présentant une perte auditive (figure 5b) et chez le même sujet acouphénique après l'écoute du signal adapteur-équaliseur pendant une durée suffisante (figure 5c). Sur ces figures, les repères 1 représentent les neurones organisés de façon tonotopique (à une position donnée correspond une bande de fréquence réduite). Sur la figure 5b, les neurones correspondant à la bordure de la perte auditive 4 répondent aux fréquences adjacentes : une zone de fréquence 2 se retrouve donc "sur-représentée" au niveau central (cette zone correspond à la fréquence de l'acouphène). Comme représenté sur la figure 5c, le signal adapteur-équaliseur 5 inverse alors les mécanismes de plasticité induits par la perte auditive (normalisation de l'organisation tonotopique centrale, repère 3).

La quatrième phase opératoire du procédé selon l'invention et par incidence, une caractéristique d'un générateur permettant la mise en oeuvre dudit procédé (cf. étape D) consiste en la génération du signal auditif ou en la fixation de ce signal ou de ses caractéristiques sur un support de stockage à des fins de restitution ultérieure. En effet, une fois les caractéristiques du signal adapteur-équaliseur calculées, ce signal peut être, selon le choix de l'utilisateur, généré directement sous sa forme temporelle (par multiplication de l'enveloppe spectrale avec le spectre d'un bruit blanc, puis transformation de Fourier inverse) à des fins d'utilisation (par exemple par transduction électroacoustique) directe ou à des fins de stockage au sein d'un support de type digital ou analogique, choisis par exemple parmi les disques compacts, les mini-disques, les cassettes, les disques durs, ou les disquettes, cette utilisation ou ce stockage étant obtenus par des moyens appropriés.

Les caractéristiques du signal peuvent également être utilisées pour programmer un dispositif électronique inclus dans ces moyens (par exemple, une mémoire programmable contenant les coefficients caractéristiques de la fonction de transfert.d'un filtre numérique, lesquels coefficients pourront être calculés notamment par transformation en Z) destiné à participer à la restitution du signal par un appareil (par exemple, une prothèse auditive ou un appareil de génération de signaux acoustiques monté en contour d'oreille ou intra-auriculaire).

Selon une caractéristique avantageuse de l'invention, le procédé selon l'invention comporte une phase opératoire finale (cf. étape E) qui consiste à ajuster en interaction avec le sujet, l'intensité du signal adapteur-équaliseur encore appelé signal correctif. Ce dernier ne doit pas masquer totalement l'acouphène et être d'une intensité suffisante pour optimiser son efficacité.

Le niveau minimal de masquabilité de l'acouphène est recherché automatiquement au moyen d'une procédure ascendante en vertu de laquelle le niveau du signal est progressivement accru de façon à éviter le phénomène d'inhibition résiduelle. Une fois le seuil de masquage obtenu, le niveau du signal est ajusté avantageusement entre 5 et 10 dB en dessous de celui-ci.

La présente invention telle que décrite précédemment offre de multiples avantages ; en particulier, elle permet d'adapter de façon spécifique le signal à la perte auditive de chaque patient souffrant d'acouphène. Les composantes utiles du signal (celles situées dans la zone de perte auditive du sujet) étant amplifiées par rapport à celles qui tombent en dehors de la perte, le niveau de stimulation peut être moindre que celui utilisé avec les appareils qui génèrent des signaux qui ne sont pas aussi précisément adaptés à l'audition des sujets. Cette propriété confère au signal adapteur-équaliseur un plus grand confort d'écoute. En effet, plus le niveau du signal est faible, moins les individus sont gênés dans leurs activités quotidiennes. De plus, la souplesse conférée par la possibilité de stocker le signal sur tout support que les patients peuvent écouter au moyen d'un baladeur ou d'une chaîne stéréophonique à leur domicile ou à l'extérieur, procure un grand confort d'utilisation à ces patients généralement habitués à des approches plus contraignantes.

**Revendications**

1. Procédé de programmation d'un générateur de signaux auditifs en vue d'atténuer, voire de supprimer les acouphènes, dans lequel on réalise les phases opératoires suivantes :

   a) mesures des seuils absolus d'audition d'un sujet,
   b) calcul sur la base desdites mesures obtenues, des caractéristiques d'un signal auditif adapté au cas particulier d'un sujet,
   le procédé étant **caractérisé en ce que** ledit calcul des caractéristiques du signal auditif est réalisé à l'aide d'algorithmes utilisant la formule suivante:

$$Ej = B + Mj - Qr\,(Aj)$$

   dans laquelle B est le niveau minimal dudit signal auditif, Mj = N- Aj avec N étant la moyenne des seuils minimaux obtenus,
   Aj sont les seuils absolus d'audition à des fréquences Fj, et
   Qr (Aj) est un facteur de correction,
   le procédé comprenant en outre une étape dans laquelle on programme le générateur de signaux auditifs de manière à ce qu'il délivre un signal auditif ayant les caractéristiques ainsi calculées.

2. Procédé selon la revendication précédente dans lequel les caractéristiques dudit signal auditif sont telles qu'elles compensent une perte auditive déterminée à partir desdits seuils absolus d'audition.

3. Procédé selon l'une des revendications 1 ou 2, comprenant en outre une étape intermédiaire entre les étapes principales a et b correspondant à la mesure de la fréquence perçue de l'acouphène.

4. Procédé selon l'une des revendications 1 à 3, dans lequel, pour réaliser ladite mesure des seuils absolus d'audition, des premiers moyens émettent dans l'oreille droite ou gauche d'un sujet un son intermittent d'intensité et de fréquences données dont on fait varier l'intensité et la fréquence en fonction des réponses données par ledit sujet au moyen d'un dispositif de réponse, ledit sujet ayant pour tâche d'indiquer s'il a entendu ou non ledit son intermittent.

5. Procédé selon l'une des revendications 1 à 4, dans lequel, pour réaliser ladite mesure de la fréquence perçue de l'acouphène, des seconds moyens émettent dans les oreilles droite et/ou gauche du sujet un son intermittent de fréquence donnée dont on fait varier la fréquence en fonction des réponses données par ledit sujet au moyen d'un dispositif de réponse, ledit sujet ayant pour tâche d'indiquer si ledit son intermittent est plus aigu, plus grave ou de mme tonalité que l'acouphène.

6. Procédé selon l'une quelconque des revendications 1 à 5, dans lequel on réalise une dernière phase opératoire qui consiste à ajuster l'intensité dudit signal correctif de manière à optimiser son efficacité.

7. Générateur de signaux auditifs comprenant des premiers moyens appropriés pour lui permettre de délivrer un signal auditif, comportant un dispositif de multiplication de l'enveloppe spectrale avec le spectre d'un bruit blanc et de transformation de Fourier inverse, à des fins d'utilisation directe ou à des fins de stockage au sein d'un support, le générateur étant **caractérisé en ce que** les premiers moyens sont agencés pour que le générateur de signaux auditifs délivre un signal auditif ayant les caractéristiques calculées dans le procédé selon l'une des revendications précédentes.

8. Générateur selon la revendication précédente dans lequel les premiers moyens comprennent un dispositif électronique programmable programmé de sorte que le générateur délivre le signal auditif.

9. Générateur selon la revendication 7, dans lequel ledit support de stockage est de type digital ou analogique.

10. Générateur selon la revendication 8, dans lequel ledit dispositif électronique est une mémoire programmable contenant des coefficients caractéristiques de la fonction de transfert d'un filtre numérique.

11. Générateur selon la revendication précédente, dans lequel lesdits coefficients sont calculés par transformation en Z.

12. Générateur selon l'une des revendications 10 ou 11, dans lequel ladite mémoire programmable est intégrée dans une prothèse auditive ou dans un appareil monté en contour d'oreille ou intra-auriculaire.

13. Générateur selon l'une quelconque des revendications 7 à 12, comprenant des moyens agencés pour ajuster, à chaque fréquence, le niveau dudit son intermittent de sorte que ledit niveau soit approximativement de même intensité perçue que l'acouphène.

**Claims**

1. Method for programming an auditory signal generator with a view to alleviating or even eliminating tinnitus, in which method the following operational phases are performed:

    a) measurements of the absolute auditory thresholds of a subject,
    b) calculation, on the basis of said measurements obtained, of the characteristics of an auditory signal adapted to the particular case of a subject,
    the method being **characterized in that** said calculation of the characteristics of the auditory signal is performed with the aid of algorithms using the following formula:

$$Ej = B + Mj - Qr (Aj)$$

    in which
    B is the minimum level of said auditory signal,
    Mj = N - Aj, with N being the average of the minimum thresholds obtained,
    Aj are the absolute auditory thresholds at frequencies Fj, and
    Qr (Aj) is a correction factor,
    the method additionally comprising a step in which the auditory signal generator is programmed in such a way that it delivers an auditory signal having the characteristics thus calculated.

2. Method according to the preceding claim, in which the characteristics of said auditory signal are such that they compensate for an auditory loss determined on the basis of said absolute auditory thresholds.

**3.** Method according to either of Claims 1 and 2, additionally comprising an intermediate step between the main steps a and b, corresponding to the measurement of the perceived frequency of the tinnitus.

**4.** Method according to one of Claims 1 to 3, in which, in order to perform said measurement of the absolute auditory thresholds, first means emit into the right or left ear of a subject an intermittent sound of given intensity and frequencies, of which the intensity and frequency are varied depending on the responses given by said subject using a response device, said subject having the task of indicating whether or not he has heard said intermittent sound.

**5.** Method according to one of Claims 1 to 4, in which, in order to perform said measurement of the perceived frequency of the tinnitus, second means emit into the right and/or left ear of the subject an intermittent sound of given frequency, the frequency of which is varied depending on the responses given by said subject using a response device, said subject having the task of indicating whether said intermittent sound is higher pitched, lower pitched or of the same tonality as the tinnitus.

**6.** Method according to any one of Claims 1 to 5, in which a final operational phase is performed that involves adjusting the intensity of said corrective signal in such a way as to optimize its effectiveness.

**7.** Auditory signal generator comprising first means allowing it to deliver an auditory signal, having a device for multiplication of the spectral envelope with the spectrum of a white noise and for inverse Fourier transform, for purposes of direct use or for purposes of storage within a support, the generator being **characterized in that** the first means are designed such that the auditory signal generator delivers an auditory signal having the characteristics calculated in the method according to one of the preceding claims.

**8.** Generator according to the preceding claim, in which the first means comprise a programmable electronic device programmed in such a way that the generator delivers the auditory signal.

**9.** Generator according to Claim 7, in which said storage support is of the digital or analog type.

**10.** Generator according to Claim 8, in which said electronic device is a programmable memory containing coefficients characteristic of the transfer function of a numeric filter.

**11.** Generator according to the preceding claim, in which said coefficients are calculated by Z-transform.

**12.** Generator according to either of Claims 10 and 11, in which said programmable memory is integrated in a hearing aid or in a device mounted around the ear or in an intra-auricular device.

**13.** Generator according to any one of Claims 7 to 12, comprising means designed to adjust the level of said intermittent sound at each frequency in such a way that said level is of approximately the same perceived intensity as the tinnitus.

**Patentansprüche**

**1.** Verfahren zur Programmierung eines Hörsignalerzeugers zur Verstärkung sowie zur Unterdrückung von Ohrgeräuschen, in welchem die folgenden Arbeitsphasen ausgeführt werden:

a) Messungen von absoluten Hörempfindlichkeiten eines Subjekts,
b) Berechnen von Kennlinien eines Hörsignals auf der Basis der erhaltenen Messungen, angepasst an den speziellen Fall eines Subjekts, wobei das Verfahren **dadurch gekennzeichnet ist, dass** die Berechnung der Kennlinien des Hörsignals mithilfe von Algorithmen durchgeführt wird, welche die folgende Formel nutzen:

$$Ej = B + Mj - Qr\,(Aj)$$

in welcher B das minimale Niveau des Hörsignals ist,
Mj = N - Aj, wobei N der Mittelwert der erhaltenen minimalen Empfindlichkeiten ist,
Aj die absoluten Hörempfindlichkeiten bei den Frequenzen Fj sind, und
Qr (Aj) ein Korrekturfaktor ist,
wobei das Verfahren ferner einen Schritt umfasst, in welchem der Hörsignalerzeuger in der Weise programmiert wird, dass dieser ein Hörsignal mit den so berechneten Kennlinien liefert.

**2.** Verfahren nach dem vorhergehenden Anspruch, in welchem die Kennlinien des Hörsignals solche sind, die ausgehend von den absoluten Hörempfindlichkeiten einen bestimmten Hörverlust kompensieren.

**3.** Verfahren nach einem der Ansprüche 1 oder 2, mit ferner einem Zwischenschritt zwischen den Hauptschritten a und b, welcher der Messung der von einem Ohrgeräusch wahrgenommenen Frequenz entspricht.

**4.** Verfahren nach einem der Ansprüche 1 bis 3, in welchem, um die Messung der absoluten Hörempfindlichkeiten durchzuführen, eine erste Einrichtung in das rechte oder linke Ohr eines Subjekts einen Ton mit intermittierender Intensität und gegebenen Frequenzen sendet, bei dem die Intensität und die Frequenz in Abhängigkeit von gegebenen Reaktionen durch das Subjekt mithilfe einer Reaktionseinrichtung variiert werden, wobei das Subjekt zur Aufgabe hat, anzuzeigen, wenn es den intermittierenden Ton verstanden hat oder nicht.

5. Verfahren nach einem der Ansprüche 1 bis 4, in welchem, um die Messung der von einem Ohrgeräusch wahrgenommenen Frequenz durchzuführen, eine zweite Einrichtung in das rechte und/oder linke Ohr des Subjekts einen intermittierenden Ton von gegebener Frequenz sendet, von dem die Frequenz in Abhängigkeit von gegebenen Reaktionen des Subjekts mithilfe einer Reaktionseinrichtung variiert wird, wobei das Subjekt zur Aufgabe hat, anzuzeigen, ob der intermittierende Ton sehr hoch, sehr tief oder in der gleiche Tonalität wie das Ohrgeräusch ist.

6. Verfahren nach einem der Ansprüche 1 bis 5, in welchem eine letzte Arbeitsphase durchgeführt wird, die darin besteht, die Intensität des Korrektursignals so einzustellen, dass sein Wirkungsgrad optimiert wird.

7. Hörsignalerzeuger mit einer ersten geeigneten Einrichtung, mit welcher diesem erlaubt wird, ein Hörsignal zu liefern, mit einer Einrichtung zur Verstärkung der spektralen Hüllkurve mit dem Spektrum eines weißen Rauschens und einer inversen Fourier Transformation, mit dem Ziel einer direkten Nutzung oder mit dem Ziel einer Speicherung innerhalb eines Trägers, wobei der Erzeuger **dadurch gekennzeichnet, ist, dass** die erste Einrichtung so angeordnet ist, dass der Erzeuger der Hörsignale ein Hörsignal mit Kennlinien liefert, die in dem Verfahren gemäß einem der vorhergehenden Ansprüche berechnet wurden.

8. Erzeuger nach dem vorstehenden Anspruch, in welchem die erste Einrichtung eine programmierbare elektronische Einrichtung umfasst, die derart programmiert ist, dass der Erzeuger das Hörsignal liefert.

9. Erzeuger nach Anspruch 7, in welchem der Träger zur Speicherung von digitaler oder analoger Bauart ist.

10. Erzeuger nach Anspruch 8, in welchem die elektronische Einrichtung ein programmierbarer Speicher ist, der für die Übertragungsfunktion eines numerischen Filters charakteristische Koeffizienten aufweist.

11. Erzeuger nach dem vorhergehenden Anspruch, in welchem die Koeffizienten durch eine Z-Transformation berechnet werden.

12. Erzeuger nach einem der Ansprüche 10 oder 11, in welchen der programmierbare Speicher in eine Hörprothese oder in einem in die Kontur eines Ohres oder intraaurikular montierten Gerät integriert ist.

13. Erzeuger nach einem der Ansprüche 7 bis 12, mit Mitteln, die so angeordnet sind, dass bei jeder Frequenz das Niveau des intermittierenden Tons derart eingestellt werden kann, dass das Niveau ungefähr gleich der als Ohrgeräusch wahrgenommenen Intensität ist.

**Etape A**
Audiométrie tonale
( mesure des seuils absolus d'audition)

**Etape B**
Acouphénométrie.
( mesure de la hauteur tonale de l'acouphène)

**Etape C**
Calcul des caractéristiques du
signal équaliseur adapteur d'acouphène

**Etape D**
Génération du signal équaliseur-adapteur d'acouphène, ou stockage
du signal ou de ses caractéristiques à des fins de génération ultérieure

**Etape E**
Ajustement du niveau
du signal équaliseur-adapteur d'acouphène

FIG.1

FIG. 2

Amplitude

Système nerveux central

Activité neuronale avec le signal adapteur.
équaliseur

Activité neuronale spontanée

Amp.

Nerf auditif

Activité neuronale avec le signal adapteur-
équaliseur

Activité neuronale spontanée

dB

Filtre cochléaire

Seuil auditif

Amp.

Entrée du système auditif

Signal adapteur. équaliseur

Acouphène

Fréquence

FIG. 3

FIG.4

EP 1 352 543 B1

FIG.5a

Seuil absolu

Fréquence

1

FIG.5b

Seuil absolu

Fréquence

2

4

FIG.5c

Seuil absolu

Fréquence

3

5

4

FIG.5

**RÉFÉRENCES CITÉES DANS LA DESCRIPTION**

**Documents brevets cités dans la description**

- US 4222393 A
- DE 29706812

- WO 9723117 A